# EUROPEAN PATENT APPLICATION

(11) **EP 4 509 055 A1**
(43) Date of publication of application: **19.02.2025**
(21) Application number: 24194495.8
(22) Date of filing: 14.08.2024
(51) Int. Cl.: A61B 6/00

(54) **MOBILE MEDICAL IMAGING APPARATUS AND MOVING TROLLEY THEREOF**

(30) Priority: 15.08.2023 CN 202311032276
(71) Applicant: Shanghai United Imaging Healthcare Co., Ltd., Shanghai 201807 (CN)
(72) Inventor: ZHANG, Chengzhe, Shanghai, 201807 (CN); TANG, Yang, Shanghai, 201807 (CN); SHI, Bo, Shanghai, 201807 (CN); XIE, Lu, Shanghai, 201807 (CN)
(74) Representative: Murgitroyd & Company

(57) **Abstract**

A mobile medical imaging apparatus and a moving trolley (100) thereof are disclosed. The mobile medical imaging apparatus has an imaging device (200) mounted on the moving trolley (100). The moving trolley includes a trolley body (10), a force detection unit (20) and a control unit (90). The trolley body (10) includes a power-assisted travelling mechanism (11). The force detection unit (20) is arranged on the trolley body (10) and configured to detect force information on a driving force applied to the trolley body (10) by an operator. The force information on the driving force includes at least a direction of the driving force. The control unit (90) is connected to the force detection unit (20) and configured to acquire the force information, and control, based on the force information, the power-assisted travelling mechanism (11) to move relative to ground.

## Description

### CROSS-REFERENCE TO RELATED APPLICATIONS

The present application claims priority to Chinese patent application No. 2023110322763, filed on August 15, 2023, the entire content of which is incorporated herein by reference.

### TECHNICAL FIELD

The present disclosure relates to the field of medical devices, and in particular, to a mobile medical imaging apparatus and a moving trolley thereof.

### BACKGROUND

Movable X-ray machines are mainly used in orthopedics, urology, endoscopy, plastic surgery, angiography, cardiology, vascular and neurology, as well as intensive care, and accident and emergency department. The movable X-ray machines are suitable for daily diagnosis in clinical environments. The conventional movable X-ray machines generally have a large weight. Depending on the power and optional functions, they are mostly between 250 kg and 400 kg, and need to be manually transported between different operating rooms. For most hospitals, the ground may be paved with floor adhesive such as epoxy flooring or PVC. Though the heavy X-ray machine may be mounted on a wheeled trolley, the rolling resistance for the wheeled trolley is very large when the wheeled trolley travels on a soft ground. The rolling resistance may even exceed 100 N. It brings great difficulties to the transportation of the X-ray machine and fine adjustment of the position of the X-ray machine, and also limits the further integration of the machine, and the addition of auxiliary functions.

### SUMMARY

The present disclosure provides a mobile medical imaging apparatus and a moving trolley thereof, to solve the problem that a conventional mobile medical imaging apparatus is difficult to be transported between different operating rooms by an operator due to its large self-weight.

A first aspect of present disclosure provides a moving trolley for a mobile medical imaging apparatus having an imaging device mounted on the moving trolley. The moving trolley may include a trolley body, a force detection unit and a control unit. The trolley body may include a power-assisted travelling mechanism. The force detection unit may be arranged on the trolley body and configured to detect force information on a driving force applied to the trolley body by an operator. The force information on the driving force may include at least a direction of the driving force. The control unit may be connected to the force detection unit and configured to acquire the force information, and control, based on the force information, the power-assisted travelling mechanism to move relative to ground.

In the first aspect, the power-assisted travelling mechanism may include a driving wheel. The driving wheel may be rotatable around a horizontal rotation axis and may be swivelable around a vertical swivel axis. The driving wheel may be configured to be independently drivable to drive the moving trolley to move in a longitudinal direction of the moving trolleyor steer relative to the longitudinal direction of the moving trolley. The moving trolley may further include an orientation detection unit configured to determine an orientation of the driving wheel based on a steering angle of the driving wheel relative to the longitudinal direction of the moving trolley, and transmit the determined orientation of the driving wheel to the control unit.

In the first aspect, the control unit may be configured to control at least one of a rotation direction or a rotation speed of the driving wheel based on the orientation of the driving wheel received from the orientation detection unit.

In the first aspect, the orientation detection unit may include a trigger member and a plurality of microswitches arranged around the trigger member, the trigger member may be swivelable around its swivel axis, the trigger member may be configured to swivel synchronously with the swiveling of the driving wheel, and to be able to touch at least one of the plurality of microswitches to trigger the at least one of the plurality of microswitches during swiveling within a predetermined angle range.

In the first aspect, the trolley body may further include a driving wheel steering linkage, and the trigger member may be connected to the driving wheel through the driving wheel steering linkage so that the trigger member may be able to swivel synchronously with the swiveling of the driving wheel.

In the first aspect, the orientation of the driving wheel may include a transverse orientation and a longitudinal orientation. When the driving wheel is in the transverse orientation, the rotation axis of the driving wheel may be parallel to the longitudinal direction of the moving trolley. When the driving wheel is in the longitudinal orientation, the rotation axis of the driving wheel may be parallel to a transverse direction of the moving trolley.

In the first aspect, the driving wheel may include a first driving wheel and a second driving wheel spaced in a transverse direction of the moving trolley. Each of the first and second driving wheels may be independently drivable to selectively rotate in a clockwise or counterclockwise direction. When each of the first and second driving wheels is in the longitudinal orientation, the control unit may be configured to control the first and second driving wheels to rotate in a same direction and at a same speed, or to control the first and second driving wheels to rotate in opposite directions and at different speeds. When each of the first and second driving wheels is in the transverse orientation, the control unit may be configured to control the first and second driving wheels to rotate in the same direction and at the same speed.

In the first aspect, the trolley body may further include a base. The driving wheel may be mounted on a bottom surface of the base, and the swivel axis of the driving wheel may be perpendicular to the bottom surface of the base. The power-assisted travelling mechanism may further include a motor configured to drive the driving wheel to rotate around the rotation axis.

In the first aspect, the trolley body may further include a non-driving wheel mounted on the bottom surface of the base. The non-driving wheel may be spaced from the driving wheel in the longitudinal direction of the moving trolley. The non-driving wheel may include a universal wheel.

In the first aspect, the motor may be incorporated into a hub of the driving wheel to form a hub motor.

In the first aspect, the motor may be mounted on the base. The power-assisted travelling mechanism may further include a transmission member. The motor may be drivingly connected to the driving wheel through the transmission member, and the motor and the transmission member may be swivelable about the swivel axis of the driving wheel in synchronism with the steering of the driving wheel.

In the first aspect, the trolley body may further include a stand and a pushing handle connected to the stand. The pushing handle may be configured to receive the driving force applied to the trolley body by the operator. The force detection unit may be arranged at a connection between the pushing handle and the stand.

In the first aspect, the trolley body may further include a steering handle connected to the stand. The steering handle may be drivingly connected to the driving wheel and configured to adjust a steering angle of the driving wheel relative to a longitudinal direction of the moving trolley.

In the first aspect, the trolley body may further include an anti-false triggering unit connected to the control unit, the anti-false triggering unit may be arranged on the pushing handle, and configured to generate an activation signal when the anti-false triggering unit is touched or pressed by the operator, and transmit the activation signal to the control unit. The control unit may be configured to acquire the force information after receiving the activation signal from the anti-false triggering unit, and control, based on the force information, the power-assisted travelling mechanism to move.

In the first aspect, the anti-false triggering unit may include at least one of a mechanical pressing component or a touch sensor.

In the first aspect, the force information on the driving force may further include a magnitude of the driving force. The control unit may be configured to control the power-assisted travelling mechanism to generate a motion adapted to the force information when the magnitude of the driving force in the force information is greater than a predetermined threshold.

In the first aspect, the moving trolley may further include an electric power storage device mounted on the vehicle body and electrically connected to the power-assisted travelling mechanism. The electric power storage device may be configured to be rechargeable by an external power source and to supply power to the power-assisted travelling mechanism.

A second aspect of the present disclosure provides a mobile medical imaging apparatus. The mobile medical imaging apparatus may include the moving trolley according to the first aspect and an imaging device mounted on the moving trolley.

In the second aspect, the imaging device may be a C-arm X-ray device.

### BRIEF DESCRIPTION OF THE DRAWINGS

The above and other aspects, features, and advantages of the present disclosure will be more apparent from the following detailed description taken in conjunction with the accompanying drawings, in which:
FIG. 1 illustrates a mobile medical imaging apparatus according to an embodiment of the present disclosure;
FIG. 2 illustrates a force detection unit and an orientation detection unit in the moving trolley according to an embodiment of the present disclosure;
FIG. 3 illustrates a motor and a rear wheel in the moving trolley according to an embodiment of the present disclosure;
FIG. 4 illustrates the rear wheel in the moving trolley with the rear wheel being in a longitudinal orientation according to an embodiment of the present disclosure;
FIG. 5 illustrates the rear wheel in the moving trolley with the rear wheel being in a transverse orientation according to an embodiment of the present disclosure; and
FIG. 6 is a flow diagram illustrating a controlling process of the moving trolley according to an embodiment of the present disclosure.

### DETAILED DESCRIPTION OF THE EMBODIMENTS

The advantages and features of the present disclosure will become apparent by referring to embodiments of the present disclosure as described below in detail in conjunction with the accompanying drawings. However, the present disclosure is not limited to the embodiments set forth below, but may be implemented in various different forms, as one of ordinary skill in the art would know. The following embodiments are provided only to inform skilled persons in the art of the scope of the present disclosure, and the present disclosure is limited only by the scope of the appended claims.

In the present disclosure, unless otherwise clearly specified and limited, the terms "first" and "second" are used for descriptive purposes only, and cannot be interpreted as indicating or implying relative importance or implicitly specifying the quantity of indicated technical features. Thus, the features defined as "first" and "second" may explicitly or implicitly include at least one of these features. In the description of the present disclosure, "plurality" means at least two, such as two, three, etc., unless otherwise clearly and specifically defined.

Terminology used herein is for the purpose of describing particular embodiments only and is not intended to be limiting of the present disclosure. For example, as used herein, the singular forms "a", "an" and "the" are intended to include the plural forms as well, unless the context clearly indicates otherwise. It will be further understood that the terms "includes" and/or "including," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

It will be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments.

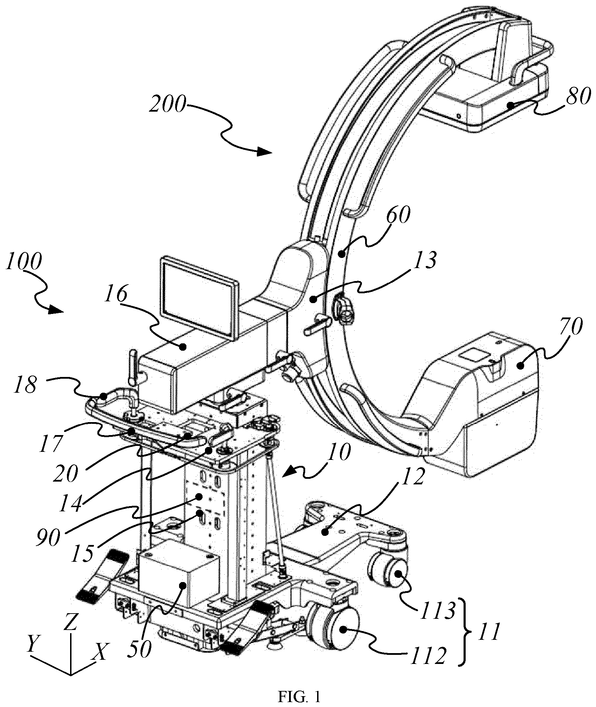

FIG. 1 illustrates a mobile medical imaging apparatus according to an embodiment of the present disclosure. As shown in FIG. 1, the mobile medical imaging apparatus includes a moving trolley 100 and an imaging device 200 mounted on the moving trolley 100. The moving trolley 100 can carry the imaging device 200 to move the imaging device 200 from one location to another location, for example, between different operating rooms. In this embodiment, the mobile medical imaging apparatus is a mobile X-ray machine, which may be used in orthopedics, urology, endoscopy, plastic surgery, angiography, cardiology, vascular and neurology, as well as intensive care, and accident and emergency department.

In this embodiment, the imaging device 200 is a C-arm X-ray device. The imaging device 200 includes a C-shaped arm 60, a detector 80 and a transmitter 70. The C-shaped arm 60 is connected to the moving trolley 100 through a support 13. The detector 80 and the transmitter 70 are respectively arranged at two opposite ends of the C-shaped arm 60. The transmitter 70 may emit a ray beam such as an X-ray beam (in this case, the transmitter 70 is an X-ray tube). The ray beam passes through an object to be detected (between both ends of the C-shaped arm 60) located on a path of the ray beam. The detector 80 receives the beam passing through the object to be detected and generates electrical signals for medical imaging.

The moving trolley 100 includes a trolley body 10. The trolley body 10 includes a stand 14, a lifting device 15, a base 12, and a connecting arm 16. The connecting arm 16 is mounted on the stand 14. An end of the connecting arm 16 is connected to the support 13 to mount the C-shaped arm 60 on the trolley body 10. The stand 14 is connected to the base 12 through the lifting device 15. The lifting device 15 is configured to adjust the position of the trolley body 10 with respect to the base 12 in a vertical direction. The base 12 is substantially parallel to the ground. That is, the lifting device 15 is configured to adjust the height of the trolley body 10 relative to the ground, and further to adjust the height of the imaging device 200 relative to the ground.

In this context, a forward direction of the moving trolley 100 means the direction in which the front of the moving trolley 100 (that is, the portion where the imaging device 200 is mounted) faces, a backward direction of the moving trolley 100 means the direction in which the rear of the moving trolley 100 faces, a left direction of the moving trolley 100 means the "left" when viewed forward from the rear of the moving trolley 100, and a right direction of the moving trolley 100 means the "right" when viewed forward from the rear of the moving trolley 100. Other terms related to "front," "back," "left" and "right" in the context have similar meanings. Further, the front-rear direction of the moving trolley 100 may be referred to as "longitudinal direction", the left-right direction of the moving trolley 100 may be referred to as "transverse direction". In the drawings, the longitudinal direction is the X-axis direction, the transverse direction is the Y-axis direction, and the vertical direction is the Z-axis direction. The X-axis, the Y-axis, and the Z-axis are perpendicular to each other to form a spatial coordinate system.

The trolley body 10 further includes a power-assisted travelling mechanism 11 and a pushing handle 17. The pushing handle 17 is configured to receive the driving force applied to the trolley body 10 by the operator. The power-assisted travelling mechanism 11 is mounted on the base 12, and has a moving ability. The moving trolley 100 can perform a straight-line movement, a curved movement, and a turning movement (i.e., steering movement) by means of the power-assisted travelling mechanism 11. The pushing handle 17 is connected to the stand 14. The operator can grasp the pushing handle 17 to apply the driving force to the trolley body 10, so that the trolley body 10 moves forward or backward, or turns under the action of the power-assisted travelling mechanism 11.

The trolley body 10 further includes a control unit 90. The dashed box in FIG. 1 schematically shows the position of the control unit 90. The control unit 90 may be arranged at the periphery of the trolley body 10 or may be arranged inside the trolley body 10. It will be appreciated that the control unit 90 may also be arranged at other locations of the mobile trolley 100. In addition, the control unit 90 may also be arranged in the imaging device 200, that is, the trolley body and the imaging device share a control unit. The control unit 90 may be a computer device including a processor. The processor may be a central processor, an embedded processor, a microprocessor or the like.

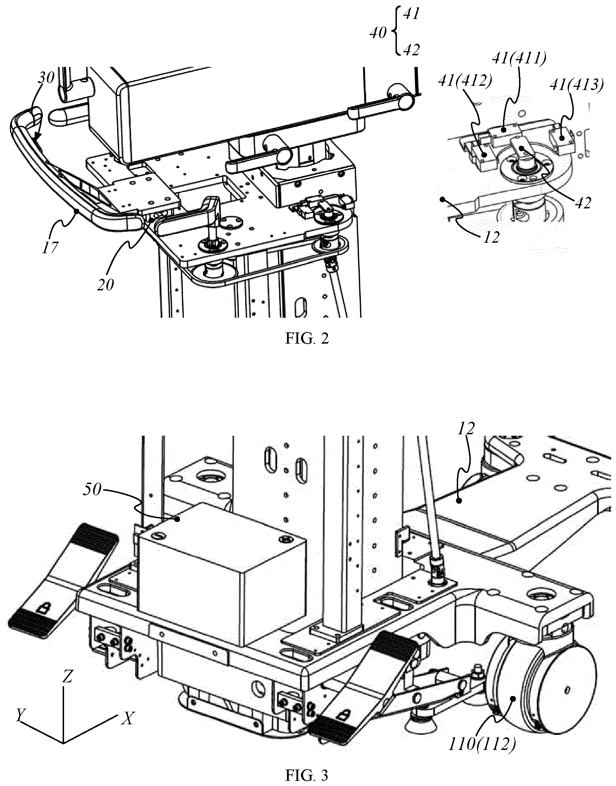

FIG. 2 illustrates a force detection unit and an orientation detection unit in the moving trolley according to an embodiment of the present disclosure. Referring to FIG. 2, the moving trolley 100 further includes a force detection unit 20. The force detection unit 20 is communicatively connected to the control unit, and the force detection unit 20 is arranged on the trolley body 10. The force detection unit 20 is configured to detect force information on the driving force applied to the trolley body 10 by the operator. The control unit 90 is configured to acquire the force information on the driving force fed back by the force detection unit 20. It can be understood that the force information on the driving force is vector information, which includes a direction of the driving force and a magnitude of the driving force. In an embodiment, the force detection unit 20 may be a force sensor arranged on a connection between the pushing handle 17 and the stand 14. That is the pushing handle 17 is connected to the stand 14 through the force sensor. The force sensor may include a strain gauge, an amplifier circuit and a filter circuit. When the operator applies the driving force to the trolley body 10 through the pushing handle 17, the strain gauge in the force sensor undergoes elastic deformation. The strain gauge generates an analog signal representing the magnitude and direction of the driving force based on its mechanical elastic deformation. The amplifier circuit and the filter circuit amplify and filter the analog signal respectively. The amplified and filtered analog signal can be converted into a digital signal. Finally, the digital signal is transmitted to the control unit 90, and the control unit 90 acquires the force information on the driving force based on the digital signal.

After acquiring the force information on the driving force, the control unit 90 controls, based on the force information on the driving force, the power-assisted travelling mechanism 11 to move relative to the ground, so that the power-assisted travelling mechanism 11 generates a motion adapted to the force information. For example, in an actual scenario, when the force detection unit 20 detects that the direction of the driving force is biased to the left, the control unit 90 controls the power-assisted travelling mechanism 11 to turn left accordingly, thereby driving the trolley body 10 to turn left. In this way, the control unit 90 controls, based on the magnitude and direction of the driving force, the power-assisted travelling mechanism 11 to operate so that the trolley body 10 moves forward or backward, or turns left or right. The cooperation relationship among the power-assisted travelling mechanism 11, the force detection unit 20, and the control unit 90 can greatly reduce the driving force to the trolley body 10 applied by the operator, facilitate the moving trolley 100 to drive the imaging device 200 to be transported between different operating rooms, and save the force applied by the operator.

The control unit 90 is configured to control the power-assisted travelling mechanism 11 to generate a motion adapted to the force information when the magnitude of the driving force in the force information is greater than a predetermined threshold. For example, only when the mechanical elastic deformation of the strain gauge of the force sensor reaches a certain degree, the control unit 90 controls the power-assisted travelling mechanism 11 to operate. In this way, the power-assisted travelling mechanism 11 can be prevented from being triggered to operate by mistake, thereby improving the safety of the apparatus.

The moving trolley 100 further includes an anti-false triggering unit 30 connected to the control unit 90. The anti-false triggering unit 30 is configured to generate an activation signal when the anti-false triggering unit 30 is touched or pressed by the operator, and transmit the activation signal to the control unit 90. The control unit 90 is configured to acquire the force information after receiving the activation signal from the anti-false triggering unit 30, and control, based on the force information, the power-assisted travelling mechanism 11 to move. The anti-false triggering unit 30 is arranged on the pushing handle 17. The anti-false triggering unit 30 may be touched or pressed while the operator grasps the pushing handle 17. Specifically, the anti-false triggering unit 30 includes at least one of a mechanical pressing component or a touch sensor. The mechanical pressing component may be, for example, a mechanical button, and the touch sensor may be, for example, a capacitive touch sensor, a resistive touch sensor, a piezoelectric touch sensor, or other type of touch sensor. The arrangement of the anti-false triggering unit 30 can improve the safety of the apparatus, to avoid erroneous triggering of the power-assisted travelling mechanism 11 due to erroneous touch of the pushing handle 17. It should be noted that when the anti-false triggering unit 30 is not activated, the operator can still control the moving trolley 100 to move by manipulating the pushing handle 17, but this consumes huge amount of physical strength and is difficult to control due to the large size and weight of the apparatus. After the anti-false triggering unit 30 generates an activation signal upon the anti-false triggering unit 30 is touched or pressed by the operator, the control unit 90 can activate the power-assisted travelling mechanism 11. With the help of the power-assisted travelling mechanism 11, it is possible to significantly reduce the driving force applied to the moving trolley 100 by the operator to realize the desired movement of the moving trolley 100, thereby facilitating the control of the operator to the movement of the mobile trolley 100.

The power-assisted travelling mechanism 11 includes a driving wheel 110. The driving wheel 110 is rotatable around a horizontal rotation axis and is swivelable around a vertical swivel axis. The driving wheel 110 is configured to be independently drivable to drive the moving trolley 100 to move in a longitudinal direction of the moving trolley 100 or steer relative to the longitudinal direction of the moving trolley 100. Referring to FIG. 1, in this embodiment, the vehicle body includes two rear wheels 112 and two front wheels 113 mounted on a bottom surface of the base 12. The two rear wheels 112 are spaced apart in the transverse direction of the moving trolley 100, and the two front wheels 113 are also spaced apart in the transverse direction of the moving trolley 100. The rear wheels 112 are spaced from the front wheels 113 in the longitudinal direction of the moving trolley 100. Each of the rear wheels 112 is the driving wheel 110 that can be driven independently to selectively rotate in either a clockwise direction (also referred to as a positive direction) or a counterclockwise direction (also referred to as a reverse direction). Each of 7the front wheels 113 is a non-driving wheel (also referred to as a driven wheel) that is driven to rotate in a clockwise or counterclockwise direction. The rotation axis of each rear wheel 112 and the rotation axis of each front wheel 113 are both parallel to the bottom surface of the base 12. The swivel of each rear wheel 112 around the swivel axis is controllable to be operably at a desired swivel angle. The front wheel 113 may be a universal wheel that can rotate 360 degrees horizontally. The rear wheel 112 may also rotate 360 degrees horizontally. Both the front wheels 113 and the rear wheels 112 may be movable casters. The swivel axes of the front wheels 113 and the rear wheels 112 are perpendicular to the bottom surface of the base 12. It can be understood that the number of front wheels and the number of rear wheels can be changed according to actual needs, and it is also possible to set the rear wheels as the non-driving wheels and the front wheels as the driving wheels. In addition, it is also understood that at least one of a plurality of rear wheels or front wheels may be provided as the driving wheel. For example, one of the two rear wheels is the driving wheel, and the other of the two rear wheels is the non-driving wheel.

The power-assisted travelling mechanism 11 further includes a motor configured to drive the driving wheel 110 to rotate around its rotation axis. In this embodiment, the motor is drivingly connected to the rear wheel 112 (i.e., the driving wheel 110) to drive the rear wheel 112 to rotate around its rotation axis. As described above, the rear wheel 112 is also swivelable relative to the base 12 around the swivel axis which is parallel to the Z axis. The trolley body 10 further includes a steering handle 18 drivingly connected to the rear wheel 112. The steering handle 180 is drivingly connected to the rear wheel 112 and is configured to adjust the steering angle of the rear wheel 112 relative to the longitudinal direction of the moving trolley 100. The operator can manipulate the steering handle 18 to drive the two rear wheels 112 to swivel synchronously around their respective swivel axes.

The front wheels 113, which act as the non-driving wheel, follow the movement of the rear wheels 112. Since the front wheels 113 can rotate horizontally by 360 degrees, the front wheels 113 can also be swiveled as the rear wheels 112 are swiveled. The forward, backward and turn of the moving trolley 100 can be achieved by the cooperation of the rear wheels 112 and the front wheels 113. The control unit 90 is configured to control, based on the force information on the driving force applied to the vehicle body 10 by the operator, the power-assisted travelling mechanism 11 to move relative to the ground. That is, the control unit 90 controls the power-assisted travelling mechanism 11 to generate a motion adapted to the force information. The control unit 90 may control the rotational speed of the motor so as to control the rotational speed of the rear wheel 112, and the control unit 90 may also control the rotational direction of the motor so as to control the rear wheel 112 to rotate clockwise or counterclockwise.

In an embodiment, the motor may be incorporated into a hub of rear wheel 112 (i.e., the driving wheel 110) to form a hub motor (also referred to as an in-wheel motor). In this way, a large number of transmission members can be omitted, and the structure of the moving trolley 100 can be made simpler. Based on the principle of the hub motor, it can be seen that the power system, the transmission system and the brake system of the moving trolley 100 can be integrated together by using the hub motor, the movement control of the moving trolley 100 is easier, and it can be ensured that the mobile medical imaging apparatus can be pushed by the operator by a small force in the state of soft ground and climbing slope. In addition, it is possible to achieve miniaturization and integration of the trolley body 10 by using the hub motor, to avoid encroachment on mounting space of other components inside the trolley body 10. Other functional devices can be integrated using unoccupied space, increasing the functional properties of the moving trolley 100. The hub motor may be implemented using any known hub motor, and details are not described herein. Due to the requirements on the positioning of the mobile C-arm medical imaging apparatus, the rear wheel 112 needs to be turned around its swivel axis for transverse traveling. Compared with arranging a motor external to the wheel, with the hub motor, it is possible to steer the motor together with the rear wheel 112 about the swivel axis of the rear wheel 112 by a simple structure, without the need for an additional separate drive conversion structure, and without the need for a movement space for the external motor to be steered.

FIG. 3 illustrates a motor and a rear wheel in the moving trolley according to an embodiment of the present disclosure. As shown in FIG. 3, an external motor 111 may be drivingly connected to the rear wheel 112. In this embodiment, the motor 111 is mounted to the base 12. The power-assisted travelling mechanism 11 further includes a transmission member 114 through which the motor 111 is drivingly connected to the rear wheel 112. The transmission member 114 transmits an output torque of the motor 111 to the rear wheel 112, and the motor 111 and the transmission member 114 are synchronously swivelable around the swivel axis of the rear wheel 112 followed by the rear wheel 112. Thus, it is ensured that the output torque of the motor 111 can still be continuously transmitted to the rear wheel 112 during the swiveling of the rear wheel 112 around the swivel axis. The transmission member 114 may be a synchronous belt, a gear member, or a transmission combination of the synchronous belt and the gear member.

Referring to FIGS. 3, the power-assisted travelling mechanism 11 includes a support plate 115, a driving pulley 116, a driven pulley 117, a friction pulley 118, and a wheel frame 119. The rear wheel 112 (i.e., the driving wheel 110) is mounted on the base 12 through the wheel frame 119, and the motor 111 is mounted on the support plate 115. An output shaft of the motor 111 is coaxially fixed to the drive pulley 116. The drive pulley 116 is connected to the driven pulley 117 through the transmission member 114. Both sides of the driven pulley 117 are coaxially fixed to the friction pulley 118, and the friction pulley 118 is in press contact with the rear wheel 112 to drive the rear wheel 112 to rotate in the form of rolling friction.

Referring to FIG. 1, the moving trolley 100 includes an electric power storage device 50. The electric power storage device 50 is mounted on the trolley body 10. Specifically, the electric power storage device 50 may be arranged on the base 12. The electric power storage device 50 is electrically connected to the power-assisted travelling mechanism 11 to supply power to the power-assisted travelling mechanism 11. Specifically, the electric power storage device 50 may supply power to the motor 111, so that the motor 111 has energy for operation. The electric power storage device 50 may be charged by an external power source. In addition, the moving trolley 100 may also include a cable that can be connected to the external power source through which power is directly supplied to the power-assisted travelling mechanism 11. For example, when the mobile medical imaging apparatus is in the operating room, the motor 111 may be electrically connected to the external power source by connecting a plug of a power cable of the mobile medical imaging apparatus to a socket in the operating room, thereby supplying power to the motor 111 from the external power source. When the mobile medical imaging apparatus needs to be transferred between different operating rooms, power is supplied to the motor 111 from the electric power storage device 50. The electric power storage device 50 includes a battery such as a lithium-ion battery, a lead-acid battery, a nickel-hydrogen battery, and a lithium iron phosphate battery.

Referring to FIG. 2, the moving trolley 100 further includes an orientation detection unit 40 configured to determine an orientation of the driving wheel 110 based on a steering angle of the driving wheel 110 relative to the longitudinal direction of the moving trolley 100, and transmit the determined orientation of the driving wheel 110 to the control unit 90. The control unit 90 controls at least one of a rotation direction or a rotation speed of the driving wheel 110 based on the orientation of the driving wheel 100. As mentioned above, the driving wheel 110 may be the rear wheel 112 or the front wheel 113. Hereinafter, the wheel 112 will be described as the driving wheel 110. The steering angle of the rear wheel 112 can be understood as an attitude angle of the rear wheel 112 relative to the base 12 after the rear wheel 112 has be swiveled around its swivel axis. The control unit 90 controls the rotation direction and/or the rotation speed of the rear wheel 112 based on the rotation direction and/or the rotation speed of the motor 111 for the rear wheel 112. Specifically, the orientation detection unit 40 determines the orientation of the rear wheel 112 relative to the base 12 based on the swivel angle of the rear wheel 112 around its swivel axis. The orientation of the rear wheel 112 includes a transverse orientation and a longitudinal orientation. When the rear wheel 112 is in the transverse orientation, the rotation axis of rear wheel 112 is parallel to the longitudinal direction of the moving trolley 100, and when the rear wheel 112 is in the longitudinal orientation, the rotation axis of the rear wheel 112 is parallel to a transverse direction of the moving trolley 100. The control unit 90 controls the rotation direction and the rotation speed of the motor 111 based on the orientation of the rear wheel 112 fed back by the orientation detection unit so as to control at least one of the rotation direction or the rotation speed of the rear wheel 112. In this embodiment, based on the orientation of rear wheel 112 and the force information on the driving force, it is possible to obtain the next movement trend of the moving trolley 100, and the control unit 90 further controls the rotation speed and the rotation direction of the motor 111, thereby assisting the moving trolley 100 to move forward, backward or transversely, or turn.

As shown in FIG. 2, in an embodiment, the orientation detection unit 40 includes a trigger member 42 and three microswitches 41 (for example, a first microswitch 411, a second microswitch 412 and a third microswitch 413) arranged on the base 12. The three microswitches 41 are arranged around the trigger member 42. The trigger member 42 is swivelable around its swivel axis. The second microswitch 412, the trigger member 42, and the third microswitch 413 are arranged parallel to the longitudinal direction (i.e., X-axis direction) of the moving trolley 100, and the first microswitch 411 and the trigger member 42 are arranged parallel to the transverse direction (i.e., Y-axis direction) of the moving trolley 100. The first microswitch 411, the second microswitch 412 and the third microswitch 413 are located in a circumferential path accessible when the trigger member 42 is swiveled. The trigger member 42 is fixedly connected to a rotating shaft 421, and the trigger member 42 can swivel with the rotation of the rotating shaft 421. The central axis of the rotating shaft 421 is the swivel axis of the trigger member 42. The trigger member 42 is configured to swivel synchronously with the swiveling of the driving wheel 110 (swiveling movement around its swivel axis) and to be able to touch at least one of the three microswitches during swiveling within a predetermined angle range. When the trigger member 42 touches one of the three microswitches 41, the microswitch 41 can be triggered. The orientation of the rear wheel 112 with respect to the base 12 can be determined based on the triggering of the microswitches 41 at different positions. It will be appreciated that in other embodiments, the orientation detection unit 40 may include two microswitches, or more than three microswitches, depending on actual needs.

Referring to FIG. 1, the trolley body 10 further includes a driving wheel steering linkage 19. An end of the driving wheel steering linkage 19 is drivingly connected to the driving wheel 110, and another end of the driving wheel steering linkage 19 is drivingly connected to the rotating shaft 421. In this way, the trigger member 42 is connected to the driving wheel 110 through the driving wheel steering linkage 19 to swivel in synchronism with the driving wheel 110. For example, in an initial state, the rotation axis of the driving wheel 110 is perpendicular to the longitudinal direction of the moving trolley 100, that is, the driving wheel 110 is in the longitudinal orientation, and the trigger member 42 touches the first microswitch 411. When the driving wheel 110 is swiveled around its swivel axis at a particular swivel angle relative to the longitudinal direction of the moving trolley 100, the trigger member 42 is also swiveled synchronously around its swivel axis at the particular swivel angle relative to the longitudinal direction of the moving trolley 100, and the trigger member 42 touches the second microswitch 412 or the third microswitch 413. In this embodiment, the power-assisted travelling mechanism 11 includes two driving wheels 110 (i.e., two rear wheels 112). The two driving wheels 110 can be swiveled synchronously through a linkage mechanism therebetween. The trigger member 42 is connected to one of the two driving wheels 110 through the driving wheel steering linkage 19. It will be appreciated that when the power-assisted travelling mechanism 11 includes more than two driving wheels 110, these driving wheels 110 can be swiveled synchronously, and the trigger member 42 is connected to one of these driving wheels 110 through the driving wheel steering linkage 19.

In some other embodiments, the orientation detection unit 40 may also be in a form of an angle potentiometer or an angle encoder. The angle potentiometer or the angle encoder can measure the swivel angle of the driving wheel 110, and output an electrical signal representing the swivel angle of the driving wheel 110 to the control unit 90. The angle potentiometer or angle encoder can be implemented using any known angle potentiometer or angle encoder, and details are not described herein.

The moving trolley 100 in this embodiment will be described in detail below with the rear wheel 112 as the driving wheel 110. Of course, in some other embodiments, the front wheel 113 may also be used as the driving wheel 110. As will be appreciated by skilled persons in the art that the implementation of the front wheel 113 as the driving wheel 110 may be further understood with reference to the implementation of the rear wheel 112 as the driving wheel 110.

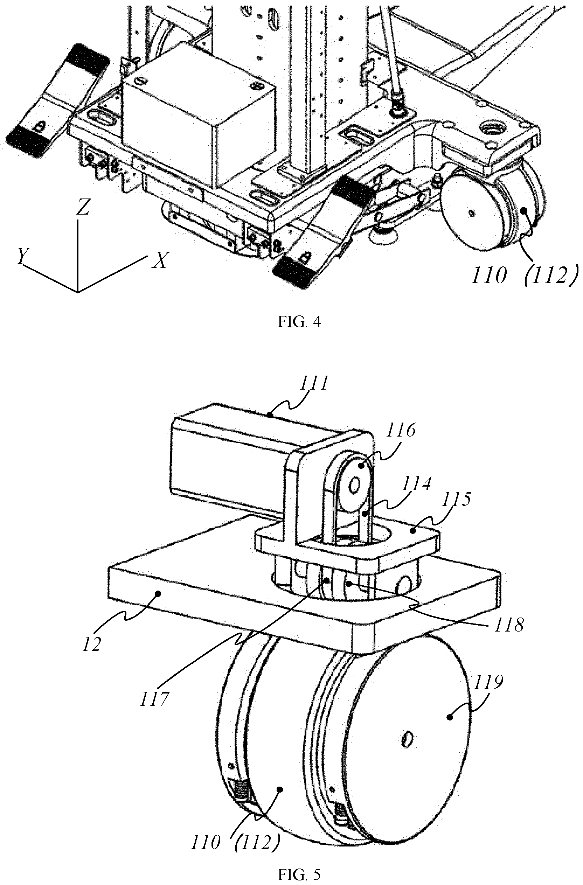

FIG. 4 illustrates the rear wheel in the moving trolley with the rear wheel being in a longitudinal orientation according to an embodiment of the present disclosure, and FIG. 5 illustrates the rear wheel in the moving trolley with the rear wheel being in a transverse orientation according to an embodiment of the present disclosure. As shown in FIG. 2, the orientation detection unit 40 includes three microswitches 41, i.e., the first microswitch 411, the second microswitch 412, and the third microswitch 413. In the longitudinal direction of the moving trolley 100, the third microswitch 413 is located in front of the trigger member 42, and the second microswitch 412 is located behind the trigger member 42. In the transverse direction of the moving trolley 100, the first microswitch 411 is located on the left of the trigger member 42. The trigger member 42 is connected to the rear wheel 112 through the driving wheel steering linkage 19. Exemplary, the state in which the rotation axis of the rear wheel 112 is perpendicular to the longitudinal direction of the moving trolley 100 is regarded as the initial state of the rear wheel 112. As shown in FIG. 4, in the initial state, the rear wheel 112 is in the longitudinal orientation. In this case, the trigger member 42 touches the first microswitch 411. The orientation detection unit 40 determines that the rear wheel 112 is in the longitudinal orientation based on the triggering of the first microswitch 411. As shown in FIG. 5, when the rear wheel 112 is swiveled around its swivel axis to be in the transverse orientation, the trigger member 42 touches the second microswitch 412 or the third microswitch 413. The orientation detection unit 40 determines that the rear wheel 112 is in the transverse orientation based on the triggering of the second microswitch 412 or the third microswitch 413.

In this embodiment, the power-assisted travelling mechanism 11 includes two rear wheels 112, and the two rear wheels 112 are swiveled synchronously. The orientation detection unit 40 can determine the common orientation of the two rear wheels 112 by determining the orientation of one of the two rear wheels 112. The orientation detection unit 40 transmits the determined orientation of the rear wheels 112 to the control unit 90. The control unit 90 controls at least one of the rotation direction or the rotation speed of each of the rear wheels 112 based on the orientation of the rear wheels 112 received from the orientation detection unit 40. For example, when the control unit 90 acquires information indicating that the rear wheels 112 are in the transverse orientation from the orientation detection unit 40, the control unit 90 may perform the following operation: controlling the motors 111 of the two rear wheels 112 to rotate in the same direction and at the same speed so that the two rear wheels 112 rotate in the same direction and at the same speed to cause the moving trolley 100 to move forward or backward in the longitudinal direction (i.e., the X-axis direction) of the moving trolley 100; or controlling the motors 111 of the two rear wheels 112 to rotate in opposite directions or at different speeds so that the two rear wheels 112 rotate in opposite directions or at different speeds to cause the moving trolley 100 to turn left or right. When the control unit 90 acquires information indicating that the rear wheels 112 are in the transverse orientation from the orientation detection unit 40, the control unit 90 controls the two motors 111 to rotate in the same direction and at the same speed so that the two rear wheels 112 rotate in the same direction and at the same speed to cause the moving trolley 100 moves in the transverse direction (i.e., the Y-axis direction) of the moving trolley 100, that is, the moving trolley 100 moves to the left or to the right.

The following describes the movement of the moving trolley 100 with the first microswitch 411 triggered, the second microswitch 412 triggered, or the third microswitch 413 triggered.

When the first microswitch 411 is triggered due to the touch of the first microswitch 411 with the trigger member 42, the orientation detection unit 40 determines that the rear wheel 112 is in the longitudinal orientation. The control unit 90 controls the rotation speeds of the motors 111 of the two wheels 112 based on the magnitude of the driving force applied by the operator, and controls the rotation directions of the motors 111 of the two wheels 112 based on the direction of the driving force. Referring to FIG. 1, the movement modes of the moving trolley 100 may include the following four modes. (1) When the direction of the driving force is detected to be forward along the X axis, the control unit 90 controls the motors 111 of the two rear wheels 112 to rotate clockwise at the same speed, so that the two rear wheels 112 respectively driven by the two motors 111 travel forward along the X axis, and the moving trolley 100 moves forward. (2) When the direction of the driving force is detected to be backward along the X axis, the control unit 90 controls the motors 111 of the two rear wheels 112 to rotate counterclockwise at the same speed, so that the two rear wheels 112 respectively driven by the two motors 111 travel backward along the X axis, and the moving trolley 100 moves backward. (3) When the direction of the driving force is detected to be left along the Y-axis, the control unit 90 controls the motor 111 corresponding to the rear wheel 112 on the left to rotate counterclockwise so that the left rear wheel 112 moves backward, and controls the motor 111 corresponding to the rear wheel 112 on the right to rotate clockwise so that the right rear wheel 112 moves forward. The whole trolley body 10 turns to the left, and the greater the magnitude of the driving force, the greater the rotation speed difference between the motors 111 of the two rear wheels 112, and the faster the speed of turning to the left. (4) When the direction of the driving force is detected to be right along the Y-axis, the control unit 90 controls the motor 111 corresponding to the rear wheel 112 on the left to rotate clockwise so that the left rear wheel 112 moves forward, and controls the motor 111 corresponding to the rear wheel 112 on the right to rotate counterclockwise so that the right rear wheel 112 moves backward. The whole trolley body 10 turns to the right, and the greater the magnitude of the driving force, the greater the rotation speed difference between the motors 111 of the two rear wheels 112, and the faster the speed of turning to the right.

When the second microswitch 412 is triggered due to the touch of the second microswitch 412 with the trigger member 42, the orientation detection unit 40 determines that the rear wheel 112 is in the transverse orientation, and more specifically, a left transverse orientation. In this case, the rotation axis of the rear wheel 112 is parallel to the X-axis, and the moving trolley 100 is restricted from moving in the X-axis direction. When the direction of the driving force is detected to be in the X-axis direction, the control unit 90 does not start the motors 111 of the two rear wheels 112. If the direction of the driving force is detected to be left along the Y-axis, the control unit 90 controls the motors 111 of the two rear wheels 112 to rotate clockwise at the same speed to drive the moving trolley 100 to move leftward along the Y-axis. If the direction of the driving force is detected to be right along the Y-axis, the control unit 90 controls the motors 111 of the two rear wheels 112 to rotate counterclockwise at the same speed to drive the moving trolley 100 to move rightward along the Y-axis.

When the third microswitch 413 is triggered due to the touch of the third microswitch 413 with the trigger member 42, the orientation detection unit 40 determines that the rear wheel 112 is in the transverse orientation, and more specifically, a right transverse orientation. In this case, the rotation axis of the rear wheel 112 is parallel to the X-axis, and the moving trolley 100 is restricted from moving in the X-axis direction. When the direction of the driving force is detected to be in the X-axis direction, the control unit 90 does not start the motors 111 of the two rear wheels 112. If the direction of the driving force is detected to be left along the Y-axis, the control unit 90 controls the motors 111 of the two rear wheels 112 to rotate counterclockwise at the same speed to drive the moving trolley 100 to move leftward along the Y-axis. If the direction of the driving force is detected to be right along the Y-axis, the control unit 90 controls the motors 111 of the two rear wheels 112 to rotate clockwise at the same speed to drive the moving trolley 100 to move rightward along the Y-axis.

In this way, by detecting which microswitch 41 is triggered, the movement trend of the moving trolley 100 can be determined, and the magnitude and direction of the driving force can be detected. With the cooperation of the power-assisted travelling mechanism 11, the moving trolley 100 can implement six types of movement, i.e., forward movement or backward movement along the X-axis, transverse movement from left to right or from right to left along the Y-axis, or turning left or right.

It can be understood that if the moving trolley only needs assistance in one orientation, then only the microswitch corresponding to the orientation may be arranged, and there is no need to microswitches corresponding to other orientations. For example, in an actual use scenario, if the power-assisted travelling mechanism 11 is required to assist the moving trolley only when the moving trolley moves forward or backward, only the first microswitch 411may be arranged to detect the longitudinal orientation of the moving trolley.

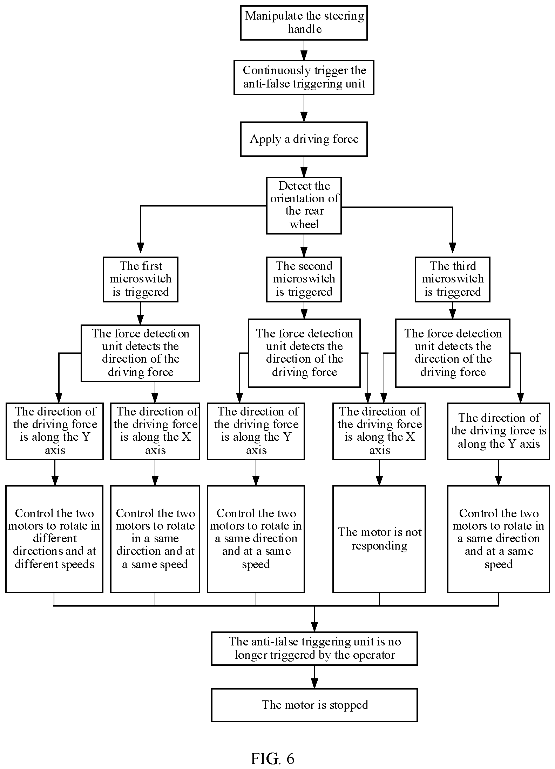

FIG. 6 is a flow diagram illustrating a controlling process of the moving trolley according to an embodiment of the present disclosure. As shown in FIG. 6, the controlling process of the moving trolley may include the following steps. In a first step, the operator manipulates the steering handle 18, causing all rear wheels 112 to swivel synchronously around their respective swivel axes. In a second step, the operator grasps the pushing handle 17 and continuously triggers the anti-false triggering unit 30. In a third step, the operator applies a driving force to the pushing handle 17. In a fourth step, the orientation detection unit 40 detects the orientation of the rear wheels 112, and the control unit 90 controls the rotation directions and the rotation speeds of the rear wheels 112. The details of the fourth step are as follows.
(1) If the first microswitch 411 is triggered, the orientation detection unit 40 determines that the rear wheel 112 is in the longitudinal orientation. When the force detection unit 20 detects that the direction of the driving force is right or left along the Y-axis, the control unit 90 controls the two motors 111 to rotate in opposite direction and at different speed, to cause the trolley body 10 to turn left or right. When the force detection unit 20 detects that the direction of the driving force is forward or backward along the X-axis, the control unit 90 controls the two motors 111 to rotate in the same direction and at the same speed, to cause the trolley body 10 to move forward or backward.
(2) If the second microswitch 412 is triggered, the orientation detection unit 40 determines that the rear wheel 112 is in the left transverse orientation. When the force detection unit 20 detects that the direction of the driving force is right or left along the Y-axis, the control unit 90 controls the two motors 111 to rotate in the same direction (clockwise direction or counterclockwise direction) and at the same speed. In this case, the trolley body 10 moves leftward or rightward along the Y-axis. When the force detection unit 20 detects that the direction of the driving force is forward or backward along the X-axis, the control unit 90 does not start the two motors 111(i.e., each motor 111 is not responding to the driving force), and the trolley body 10 is in a stationary state.
(3) If the third microswitch 413 is triggered, the orientation detection unit 40 determines that the rear wheel 112 is in the right transverse orientation. When the force detection unit 20 detects that the direction of the driving force is right or left along the Y-axis, the control unit 90 controls the two motors 111 to rotate in the same direction (clockwise direction or counterclockwise direction) and at the same speed. In this case, the trolley body 10 is moves leftward or rightward along the Y-axis. When the force detection unit 20 detects that the direction of the driving force is forward or backward along the X-axis, the control unit 90 does not start the two motors 111 (i.e., each motor 111 is not responding to the driving force), and the trolley body 10 is in a stationary state.

In a fifth step, the operator releases the pushing handle 17 so as not to touch or press the anti-false triggering unit 30, the anti-false triggering unit 30 no longer generates the activation signal. The control unit 90 stops the control of the power-assisted travelling mechanism 11, and the motor 111 stops the operation. In this case, the vehicle body 10 may continue to move when a large driving force is applied to the trolley body 10 by the operator, or the trolley body 10 may be in a stationary state.

In the mobile medical imaging apparatus and the moving trolley according to above embodiments, the moving trolley includes the trolley body, the force detection unit, and the control unit 90. The trolley body includes a power-assisted travelling mechanism. The force detection unit is arranged on the trolley body, and is configured to detect the force information on the driving force applied to the trolley body by the operator. The control unit 90 is connected to the force detection unit and configured to acquire the force information, and control the power-assisted travelling mechanism to generate a movement adapted to the force information. With such a configuration, when the force detection unit detects the driving force applied to the trolley body by the operator, the control unit 90 drives the power-assisted travelling mechanism to operate so that the power-assisted travelling mechanism generates the movement adapted to the force information on the driving force. For example, based on the magnitude and the direction of the driving force, the control unit 90 controls the power-assisted travelling mechanism to operate so that the trolley body moves forward or backward, or turns left or right. In this way, the moving trolley is able to achieve the desired movement with a reduced driving force applied to the trolley body by the operator. As a result, it is easy to transport the mobile medical imaging apparatus between different operating rooms.

Although various illustrative embodiments are described above, any of a number of changes may be made to various embodiments without departing from the scope of the present disclosure as described by the claims. The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of present disclosure. Although specific embodiments have been illustrated and described herein, any arrangement calculated to achieve the same purpose may be substituted for the specific embodiments shown. The present disclosure is intended to cover any and all adaptations or variations of various embodiments. Combinations of the above embodiments, and other embodiments not specifically described herein, will be apparent to persons skilled in the art upon reviewing the above description.

## Claims

1. A moving trolley (100) for carrying an imaging device (200), the moving trolley (100) comprising:
a trolley body (10) comprising a power-assisted travelling mechanism (11);
a force detection unit (20) arranged on the trolley body (10) and configured to detect force information of a driving force applied to the trolley body (10) by an operator, the force information of the driving force comprising at least a direction of the driving force; and
a control unit (90) connected to the force detection unit (20) and configured to acquire the force information, and control, based on the force information, the power-assisted travelling mechanism (11) to move relative to ground.

2. The moving trolley (100) of claim 1, wherein the power-assisted travelling mechanism (11) comprises a driving wheel (110), the driving wheel (110) being rotatable around a horizontal rotation axis and being swivelable around a vertical swivel axis, the driving wheel (110) being configured to be independently drivable to drive the moving trolley (100) to move in a longitudinal direction of the moving trolley (100) or steer relative to the longitudinal direction of the moving trolley (100); and
wherein the moving trolley (100) further comprises an orientation detection unit (40) configured to determine an orientation of the driving wheel (110) based on a steering angle of the driving wheel (110) relative to the longitudinal direction of the moving trolley (100), and transmit the determined orientation of the driving wheel (110) to the control unit (90).

3. The moving trolley (100) of claim 2, wherein the control unit (90) is configured to control at least one of a rotation direction or a rotation speed of the driving wheel based on the orientation of the driving wheel received from the orientation detection unit.

4. The moving trolley (100) of claim 2 or 3, wherein the orientation detection unit (40) comprises a trigger member (42) and a plurality of microswitches (41) arranged around the trigger member (42), the trigger member (42) is swivelable around its swivel axis, the trigger member (42) is configured to swivel synchronously with the swiveling of the driving wheel (110), and to be able to touch at least one of the plurality of microswitches to trigger the at least one of the plurality of microswitches during swiveling within a predetermined angle range.

5. The moving trolley (100) of claim 4, wherein the trolley body (10) further comprises a driving wheel steering linkage (19), and the trigger member (42) is connected to the driving wheel (110) through the driving wheel steering linkage (19) so that the trigger member (42) is able to swivel synchronously with the swiveling of the driving wheel (110).

6. The moving trolley (100) of any one of claims 2 to 5, wherein the orientation of the driving wheel (110) comprises a transverse orientation and a longitudinal orientation,
when the driving wheel (110) is in the transverse orientation, the rotation axis of the driving wheel (110) is parallel to the longitudinal direction of the moving trolley (100), and
when the driving wheel (110) is in the longitudinal orientation, the rotation axis of the driving wheel (110) is parallel to a transverse direction of the moving trolley (100).

7. The moving trolley (100) of claim 6, wherein the driving wheel (110) comprises a first driving wheel and a second driving wheel spaced in a transverse direction of the moving trolley (100), each of the first and second driving wheels being independently drivable to selectively rotate in a clockwise or counterclockwise direction;
wherein when each of the first and second driving wheels is in the longitudinal orientation, the control unit (90) is configured to control the first and second driving wheels to rotate in a same direction and at a same speed, or to control the first and second driving wheels to rotate in opposite directions and at different speeds; and
wherein when each of the first and second driving wheels is in the transverse orientation, the control unit (90) is configured to control the first and second driving wheels to rotate in the same direction and at the same speed.

8. The moving trolley (100) of any one of claims 2 to 7, wherein the trolley body (10) further comprises a base (12), the driving wheel (110) is mounted on a bottom surface of the base (12), and the swivel axis of the driving wheel (110) is perpendicular to the bottom surface of the base (12);
the power-assisted travelling mechanism (11) further comprises a motor (111) configured to drive the driving wheel (110) to rotate around the rotation axis; and
optionally wherein the trolley body (10) further comprises a non-driving wheel (113) mounted on the bottom surface of the base (12), the non-driving wheel (113) be spaced from the driving wheel (110) in the longitudinal direction of the moving trolley (100), the non-driving wheel (113) comprising a universal wheel.

9. The moving trolley (100) of claim 8, wherein the motor (111) is incorporated into a hub of the driving wheel (110) to form a hub motor.

10. The moving trolley (100) of claim 8, wherein the motor (111) is mounted on the base (12); and
wherein the power-assisted travelling mechanism (11) further comprises a transmission member (114), the motor (111) is drivingly connected to the driving wheel (110) through the transmission member (114), and the motor (111) and the transmission member (114) are swivelable about the swivel axis of the driving wheel (110) in synchronism with the steering of the driving wheel (110).

11. The moving trolley (100) of any one of claims 1 to 10, wherein the trolley body (10) further comprises a stand (14) and a pushing handle (17) connected to the stand (14), the pushing handle (17) being configured to receive the driving force applied to the trolley body (10) by the operator;
wherein the force detection unit (20) is arranged at a connection between the pushing handle (17) and the stand (14); and
optionally wherein the trolley body (10) further comprises a steering handle (18) connected to the stand (14), the steering handle (18) being drivingly connected to the driving wheel (110) and configured to adjust a steering angle of the driving wheel (110) relative to a longitudinal direction of the moving trolley (100).

12. The moving trolley (100) of claim 11, wherein the trolley body (10) further comprises an anti-false triggering unit (30) connected to the control unit (90), the anti-false triggering unit (30) is arranged on the pushing handle (17), and configured to generate an activation signal when the anti-false triggering unit (30) is touched or pressed by the operator, and transmit the activation signal to the control unit (90);
wherein the control unit (90) is configured to acquire the force information after receiving the activation signal from the anti-false triggering unit (30), and control, based on the force information, the power-assisted travelling mechanism (11) to move; and
optionally wherein the anti-false triggering unit (30) comprises at least one of a mechanical pressing component or a touch sensor.

13. The moving trolley (100) of any one of claims 1 to 12, wherein the force information on the driving force further comprises a magnitude of the driving force, and the control unit (90) is configured to control the power-assisted travelling mechanism (11) to generate a motion adapted to the force information when the magnitude of the driving force in the force information is greater than a predetermined threshold.

14. The moving trolley (100) of any one of claims 1 to 13, further comprising an electric power storage device (50) mounted on the vehicle body (100) and electrically connected to the power-assisted travelling mechanism (11), the electric power storage device (50) being configured to be rechargeable by an external power source and to supply power to the power-assisted travelling mechanism (11).

15. A mobile medical imaging apparatus, comprising:
the moving trolley (100) of any one of claims 1 to 14; and
an imaging device (200) mounted on the moving trolley (100);
optionally wherein the imaging device (200) is a C-arm X-ray device.
